# EUROPÄISCHE PATENTSCHRIFT

(11) **EP 1 808 457 B1**
(45) Veröffentlichungstag und Bekanntmachung des Hinweises auf die Patenterteilung: **16.07.2008**
(21) Anmeldenummer: 06124624.5
(22) Anmeldetag: 23.11.2006
(51) Int. Cl.: C08K 5/101, C08K 5/00, C07C 69/82, C07C 67/03, C08L 27/06

(54) **Terephthalsäuredialkylester und deren Verwendung als Weichmacher**
Dialkylterephthalates and their use as plasticizers
Esters d'acides téréphthaliques utilisés comme plastifiants

(30) Priorität: 12.01.2006 DE 102006001795
(43) Veröffentlichungstag der Anmeldung: 18.07.2007
(73) Patentinhaber: Evonik Oxeno GmbH, 45772 Marl (DE)
(72) Erfinder: Grass, Michael Dr., 45721, Haltern am See (DE)
(74) Vertreter: Hirsch, Hans-Ludwig

(56) Entgegenhaltungen:
- WO-A-20/07021987
- DE-A1- 19 927 978
- US-A1- 3 224 995
- US-A1- 2004 238 787
- US-A1- 2004 260 113
- NEMATOLLAHI, JAY; GUESS, WALLACE L.; AUTIAN, JOHN: "Plasticizers in medical application. I. Analysis and toxicity evaluation of dialkyl benzenedicarboxylates" JOURNAL OF PHARMACEUTICAL SCIENCES, Bd. 56, Nr. 11, 1967, Seiten 1446-1453, XP009080785
- BEELER A D: "TEREPHTHALATE ESTERS A NEW CLASS OF PLASTICIZERS FOR POLYVINYL CHLORIDE" SPE ANNUAL TECHNICAL CONFERENCE AND EXHIBITION, 26. April 1976 (1976-04-26), Seiten 613-615, XP008040735
- DATABASE WPI Week 200421 Derwent Publications Ltd., London, GB; AN 2004-217834 XP002425475 -& JP 2003 301082 A (MITSUBISHI CHEM CORP) 21. Oktober 2003 (2003-10-21)
- DATABASE WPI Week 200142 Derwent Publications Ltd., London, GB; AN 2001-392553 XP002425476 -& JP 2001 031794 A (HOKOKU SEIYU KK) 6. Februar 2001 (2001-02-06)

## Beschreibung

Die Erfindung betrifft Terephthalsäuredialkylester mit Alkyl = Alkylreste, die eine längste Kohlenstoffkette von zumindest 4 Kohlenstoffatomen und eine Gesamtzahl an Kohlenstoffatomen pro Alkylrest von 5 aufweisen, ein Verfahren zu deren Herstellung sowie die Verwendung dieser Produkte.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP) und Diisodecylphthalat (DIDP) Verwendung finden. Mit zunehmender Kettenlänge der Ester steigen die Löse- bzw. Geliertemperaturen und somit die Verarbeitungstemperaturen des Weich-PVC an. Die Verarbeitungstemperaturen können durch Zusatz von sogenannten Schnellgelierern wie beispielsweise die kurzkettigen Phthalate Dibutylphthalat (DBP), Diisobutylphthalat (DIBP), Benzylbutylphthalat (BBP) oder Diisoheptylphthalat (DIHP) wieder reduziert werden.

Auf Grund von Diskussionen um reproduktionstoxische Effekte, die bereits in einigen Fällen zu einer verstärkten gefahrstoffrechtlichen Kennzeichnung geführt haben, muss davon ausgegangen werden, dass die Verwendung dieser kürzerkettigen Phthalate künftig deutlich zurückgehen wird. Es besteht daher Bedarf nach nicht kennzeichnungspflichtigen Weichmachern, die als Schnellgelierer Verwendung finden können und die aus Rohstoffen hergestellt werden, die weltweit in großen Mengen verfügbar sind.

Aufgabe der vorliegenden Erfindung war es deshalb eine Weichmacherzusammensetzung bereitzustellen, der als Schnellgelierer eingesetzt werden kann und der vorzugsweise bei Verwendung in Plastisolen diesen auch eine gewisse Lagerstabiltät verleiht, d. h. einen nur geringen Anstieg der Viskosität mit der Zeit bewirkt.

Neben der Phthalsäure stellt die Terephthalsäure bzw. das Derivat Dimethylterephthalat einen Stoff dar, der mit einer geschätzten Jahresproduktion im Bereich von Millionen Tonnen in großen Mengen verfügbar ist. Ein Massenprodukt ausgehend von Terephthalsäure ist beispielsweise Polyethylenterephthalat (PET). Bisher hat aber nur ein monomerer Ester der Terephthalsäure industriell als Weichmacher für PVC eine gewisse Bedeutung erlangt, nämlich Di-2-ethylhexyl-terephthalat (DEHT oder auch DOTP), der beispielsweise von der Firma Eastman Chemical vermarktet wird.

In US 5,071,690 wird die Möglichkeit beschrieben, Di-n-butylterephthalat als Weichmacher für die Herstellung von Polyesterfilmen einzusetzen. In US 6,051,305 wird die Verwendung von thermoplastischen Polymer-Partikeln, die Di-Butylterephthalat aufweisen, als Tonerpartikel beschrieben.

Don Beeler beschreibt in Soc. Plast. Eng., Tech. Pap (1976), 22 613 - 615, dass Terephthalsäureester aus Alkoholen mit 1 bis 6 Kohlenstoffatomen bis auf eine Ausnahme (Diisopropyl-terephthalat) alle Feststoffe sind und mit PVC unverträglich sind. Trotz der Aussage, dass Terephthalsäureester in ihrem Eigenschaftsbild im Wesentlichen den um ein C-Atom in der Alkoholkette längeren Phthalsäureestern entsprechen, war es daher nicht zu erwarten, dass Terephthalsäureester aus Alkoholen mit einer Kettenlänge von 4 bis 6 C-Atomen als Schnellgelierer in Frage kommen könnten.

Überraschenderweise wurde gefunden, dass Terephthalsäureester mit 4 bis 5C-Atomen in der längsten Kohlenstoffkette des Alkohols als (schnell gelierende) Weichmacher gut geeignet sind. Dies ist insbesondere überraschend, da Don Beeler beschrieben hat, dass solche Terephthalsäureester mit PVC unverträglich wären.

Gegenstand der vorliegenden Erfindung sind daher Weichmacherzusammensetzungen enthaltend Terephthalsäuredialkylester, welche dadurch gekennzeichnet sind, dass die Alkylreste eine längste Kohlenstoffkette von zumindest 4 Kohlenstoffatomen aufweisen und eine Gesamtzahl an Kohlenstoffatomen pro Alkylrest von 5 aufweisen.

Ebenfalls Gegenstand der vorliegenden Erfindung ist die Verwendung von Terephthalsäuredialkylestern als Weichmacher in Zusammensetzungen, enthaltend ein Polymer, ausgewählt aus PVC, PVB, PAMA oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril, cyclischen Olefinen, welche dadurch gekennzeichnet ist, dass die Alkylreste der Terephthalsäuredialkylester eine längste Kohlenstoffkette von zumindest 4 Kohlenstoffatomen aufweisen und dass die Alkylreste eine Gesamtzahl an Kohlenstoffatomen pro Alkylrest von 5 aufweisen.

Außerdem ist Gegenstand der vorliegenden Erfindung eine Weichmacherzusammensetzung, enthaltend einen Weichmacher und ein Polymer, ausgewählt aus PVC, PVB, PAMA oder Homo- oder Copolymere auf Basis von Ethylen, Propylen, Butadien, Vinylacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, cyclischen Olefinen, welche dadurch gekennzeichnet ist, dass die Zusammensetzung als Weichmacher einen Terephthalsäuredialkylester enthält, bei dem die Alkylreste eine längste Kohlenstoffkette von zumindest 4 Kohlenstoffatomen aufweisen und eine Gesamtzahl an Kohlenstoffatomen pro Alkylrest von 5 aufweisen, sowie ein Verfahren zur Herstellung von Terephthalsäuredialkylestern, deren Alkylreste eine längste Kohlenstoffkette von zumindest 4 Kohlenstoffatomen aufweisen und eine Gesamtzahl an Kohlenstoffatomen pro Alkylrest von 5 aufweisen, umfassend die Schritte,
a) Hydroformylierung von C₄-Olefinen unter Erhalt von C₅- Aldehyden,
b) Hydrierung der in Schritt a) erhaltenen Aldehyde zum entsprechenden Alkohol und
c) Umsetzung des Alkohols aus b) mit Terephthalsäure oder einem Derivat der Terephthalsäure zum entsprechenden Diester.

Die erfindungsgemäßen Weichmacher haben den Vorteil, dass sie bei Verwendung in Plastisolen eine gute Lagerstabilität aufweisen. Der Einsatz der erfindungsgemäßen Weichmacher ist insbesondere vorteilhaft, weil die Weichmacher eine gute Gelierung bewirken. Je nach Verwendungszweck können erfindungsgemäße Weichmacher, die einen Terephthalsäuredialkylester enthalten, bei dem die Alkylreste eine längste Kohlenstoffkette von zumindest 4 Kohlenstoffatomen aufweisen und eine Gesamtzahl an Kohlenstoffatomen pro Alkylrest von 5 aufweisen, eingesetzt werden, wobei je nach Gesamtzahl der Kohlenstoffatome im Alkylrest die Geliereigenschaften verbessert oder die Flüchtigkeit verringert wird. Die erfindungsgemäßen Weichmacher sind insbesondere zur Verwendung in Plastisolen geeignet, da sie eine niedrige Viskosität des Plastisols ermöglichen. Ein weiterer Vorteil besteht darin, dass der erfindungsgemäße Weichmacher als Ersatz von Düsobutylphthalat, Di-n-butylphthalat, Di-pentylphthalat oder Diisoheptylphthalat eingesetzt werden kann, da mit ihm vergleichbare mechanische Eigenschaften erzielt werden können. Der erfindungsgemäße Weichmacher bzw. Zusammensetzungen, die Terephthalsäuredialkylester enthalten, bei dem die Alkylreste eine längste Kohlenstoffkette von zumindest 4 Kohlenstoffatomen aufweisen und eine Gesamtzahl an Kohlenstoffatomen pro Alkylrest von 5 aufweisen, haben außerdem den Vorteil, dass sie auf Terephthalsäure und nicht auf Phthalsäure basieren. Diester der Phthalsäure, insbesondere 2-Ethylhexylphthalate, weisen gemäß James L. Cooper (im Vortrag: "An Alternative to DEHP in Plasticized PVC", auf Vinyl Formulators Divison, 16th Annual Compounding Conference, Harrah's/Harvey's Resort, Lake Tahoe, Nevada, vom 17. bis 19. Juli 2005) einen anderen Metabolismus als die Diester der Terephthalsäure auf. Beim Abbau werden die Terephthalate zunächst vollständig zu Alkohol und Terephthalsäure hydrolyisert während die Phthalate nur zum Monoester hydrolysiert werden. Diese Monoester wurden in Laborstudien als eine der toxikologisch aktiven Substanzen identifiziert. Wegen des unterschiedlichen Metabolismus von Di-2-Ethylhexylphthalat und Di-2-Ethylhexylterephthalat weist Di-2-Ethylhexylterephthalat gemäß James L. Cooper eine deutlich geringere Toxizität als Di-2-Ethylhexylphthalat auf. Da die erfindungsgemäßen Weichmacher auf Terephthalsäure basieren, kann angenommen werden, dass auch beim Abbau der erfindungsgemäß vorhandenen Terephthalate eine vollständige Hydrolyse zur Terephthalsäure erfolgen wird und die Terephthalate somit ebenfalls eine geringere Toxizität als die korrespondierenden Phthalate aufweisen.

Nachfolgend wird die Erfindung beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind nachfolgend Bereiche, allgemeine Formeln oder Verbindungsklassen angegeben, so soll die Offenbarung nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können, ohne dass diese aus Gründen der besseren Übersichtlichkeit explizit genannt worden sind.

Der erfindungsgemäße Weichmacher bzw. die erfindungsgemäße Weichmacherzusammensetzung (nachfolgend der Einfachheit halber nur als Weichmacher bezeichnet) enthaltend Terephthalsäuredialkylester, zeichnet sich dadurch aus, dass die Alkylreste eine längste Kohlenstoffkette von zumindest 4 Kohlenstoffatomen aufweisen und eine Gesamtzahl an Kohlenstoffatomen pro Alkylrest von 5 aufweisen. Die Alkylreste können in einem Estermolekül gleich oder unterschiedlich sein. Werden bei der Herstellung der Ester keine isomeren-reinen Alkohole eingesetzt, werden üblicherweise Terephthalsäuredialkylestergemische erhalten, die Estermoleküle enthalten, die unterschiedliche Alkylreste aufweisen.

Die Alkylreste der Terephthalsäuredialkylester sind vorzugsweise zu mehr als 60 % (Massen-% bezogen auf die Summe der Alkylreste) n-Pentyl-Reste. Bevorzugt sind die Alkylreste der Terephthalsäuredialkylester von 70 bis 99,9 % n-Pentyl-Reste und von 30 bis 0,1 % Methylbutyl-Reste, insbesondere 2-Methylbutylreste, besonders bevorzugt von 85 bis 98 % n-Pentyl-Reste und von 15 bis 2 % Methylbutyl-Reste, insbesondere 2-Methylbutylreste, und ganz besonders bevorzugt von 90 bis 96 % n-Pentyl-Reste und von 10 bis 4 % Methylbutyl-Reste, insbesondere 2-Methylbutylreste. Vorzugsweise sind die Methylbutyl-Reste zu mehr als 50 %, bevorzugt zu mehr als 75 % und besonders bevorzugt zu mehr als 95 % 2-Methylbutyl-Reste. Die prozentuale Verteilung der C₅-Alkyl-Reste kann auf einfache Weise durch Verseifen der Ester, Abtrennen des erhaltenen Alkohols und gaschromatographische (GC) Analyse des Alkohols erfolgen. Beispielsweise kann die gaschromatographische Trennung auf einer Polydimethylsiloxan-Säule (z. B. DB 5) als stationärer Phase mit einer Länge von 60 m, einem inneren Durchmesser von 0,25 mm und einer Filmdicke von 0,25 µm durchgeführt werden.

Es ist vorteilhaft, wenn die erfindungsgemäßen Weichmacher neben Terephthalsäuredialkylester mit einer Gesamtzahl an Kohlenstoffatomen von 5 pro Alkylrest zumindest einen primären Weichmacher aufweisen. Unter primären Weichmachern werden solche Verbindungen verstanden, die als alleinige Weichmacher eingesetzt werden können und in weiten Konzentrationsbereichen (wenige Prozent, z. B. 10 Massen-% bis hin zu Verhältnissen mit mehr Weichmacher als Polymer) mit dem weichzumachenden Polymer verträglich sind. Durch das Vorhandensein von primären Weichmachern können die Eigenschaften des erfindungsgemäßen Weichmachers variiert werden. Vorzugsweise weist der erfindungsgemäße Weichmacher neben Terephthalsäuredialkylester mit einer Gesamtzahl an Kohlenstoffatomen von 5 pro Alkylrest zumindest einen primären Weichmacher, ausgewählt aus den Phthalsäuredialkylestern, Trimellitsäuretrialkylestern, Adipinsäuredialkyl-estern, Terephthalsäuredialkylestern, 1,2-Cyclohexandisäurealkylestern, 1,3-Cyclohexandi-säurealkylestern und 1,4-Cyclohexandisäurealkylestern mit Alkyl = Alkylrest mit 7 bis 11, insbesondere 8 bis 10 Kohlenstoffatomen, den Glykoldibenzoaten und den Alkylsulfonsäureestern von Phenol mit Alkyl = Alkylrest mit 8 bis 22 Kohlenstoffatomen sowie den acylierten und nicht acylierten Citronensäuretrialkylestern, Polymerweichmachern und Glycerinestern auf.

Der Anteil an primären Weichmachern beträgt im erfindungsgemäßen Weichmacher vorzugsweise von 20 bis 99 Massen-%, bevorzugt von 25 bis 80 Massen-% und besonders bevorzugt von 30 bis 75 Massen-%, bezogen auf die Gesamtmasse aller eingesetzten Weichmacher.

Es kann vorteilhaft sein, wenn der erfindungsgemäße Weichmacher neben Terephthalsäuredialkylestern mit einer Gesamtzahl an Kohlenstoffatomen von 5 pro Alkylrest, zumindest einen Benzoesäurealkylester mit Alkyl = Alkylrest mit 7 bis 13, insbesondere 8 bis 10 Kohlenstoffatomen, vorzugsweise Benzoesäurenonyl- oder Benzoesäureisononylester und besonders bevorzugt Benzoesäureisononylester aufweist. Der Anteil an Benzoesäurealkylester mit Alkyl = Alkylrest mit 8 bis 10 Kohlenstoffatomen, insbesondere an Benzoesäureisononylester beträgt im erfindungsgemäßen Weichmacher vorzugsweise von 5 bis 90 Massen-%, bevorzugt von 10 bis 80 Massen-% und besonders bevorzugt von 30 bis 70 Massen-%.

Besonders bevorzugt weist der erfindungsgemäße Weichmacher neben Terephthalsäuredialkylestern mit einer Gesamtzahl an Kohlenstoffatomen von 5 pro Alkylrest und zumindest einem Benzoesäurealkylester mit Alkyl = Alkylrest mit 8 bis 10, bevorzugt 9 Kohlenstoffatomen, zumindest einen primären Weichmacher auf. Solche erfindungsgemäßen Weichmacher weisen vorzugsweise einen Anteil an Terephthalsäuredialkylestern mit einer Gesamtzahl an Kohlenstoffatomen von 5 pro Alkylrest von 5 bis 70 Massen-%, einen Anteil an Benzoesäurealkylester mit Alkyl = Alkylrest mit 8 bis 10 Kohlenstoffatomen von 5 bis 70 Massen-% und einen Anteil an primären Weichmachern von 20 bis 90 Massen-% auf.

Die erfindungsgemäße Verwendung von Terephthalsäuredialkylester als Weichmacher in Weichmacherzusammensetzung enthaltend ein Polymer, ausgewählt aus PVC, PVB, PAMA oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril, cyclischen Olefinen, oder in Klebstoffen, Farben oder Lacken, zeichnet sich dadurch aus, dass die Alkylreste des verwendeten Terephthalsäuredialkylesters eine längste Kohlenstoffkette von zumindest 4 Kohlenstoffatomen aufweisen und eine Gesamtzahl an Kohlenstoffatomen pro Alkylrest von 5 aufweisen.

Es kann vorteilhaft sein, wenn Terephthalsäuredialkylester als Weichmacher verwendet werden, bei denen die Alkylreste des Terephthalsäuredialkylesters eine Gesamtzahl an Kohlenstoffatomen pro Alkylrest von 5 aufweisen. Diese erfindungsgemäßen Weichmacher haben den Vorteil, dass sie gegenüber den Terephthalsäuredialkylestern, bei denen die Alkylreste eine Gesamtzahl an Kohlenstoffatomen pro Alkylrest von 4 aufweisen, eine niedrigere Flüchtigkeit aufweisen.

Vorzugsweise werden Terephthalsäuredialkylester verwendet, deren Alkylreste zu mehr als 60 % (Massen-% bezogen auf die Summe der Alkylreste) n-Pentyl-Reste sind. Unter Terephthalsäuredialkylestern werden dabei auch Mischungen von Terephthalsäuredialkylestern verstanden, die Terephthalsäuredialkylester mit unterschiedlichen Alkylresten aufweisen. Bevorzugt werden Terephthalsäuredialkylester verwendet, deren Alkylreste von 70 bis 99,9 % (Massen-% bezogen auf die Summe der Alkylreste) n-Pentyl-Reste und von 30 bis 0,1 % Methylbutyl-Reste, insbesondere 2-Methylbutylreste, besonders bevorzugt von 85 bis 98 % n-Pentyl-Reste und von 15 bis 2 % Methylbutyl-Reste, insbesondere 2-Methylbutylreste, und ganz besonders bevorzugt von 90 bis 95 % n-Pentyl-Reste und von 10 bis 5 % Methylbutyl-Reste, insbesondere 2-Methylbutylreste sind. Vorzugsweise sind die Methylbutyl-Reste zu mehr als 50 %, bevorzugt zu mehr als 75 % und besonders bevorzugt zu mehr als 95 % 2-Methylbutyl-Reste. Die vorgenannten Bereiche sind insbesondere dann bevorzugt, wenn erfindungsgemäß Terephthalsäuredialkylester verwendet werden, die eine Gesamtzahl an Kohlenstoffatomen pro Alkylrest von 5 aufweisen. Die prozentuale Verteilung der C₅-Alkyl-Reste kann auf einfache Weise durch Verseifen der Ester, Abtrennen des erhaltenen Alkohols und gaschromatographische (GC) Analyse des Alkohols erfolgen.

Die erfindungsgemäßen Weichmacher bzw. die Terephthalsäuredialkylester, die eine längste Kohlenstoffkette von zumindest 4 Kohlenstoffatomen aufweisen und eine Gesamtzahl an Kohlenstoffatomen pro Alkylrest von 5 aufweisen, können z. B. als Viskositätserniedriger und schnellgelierende Weichmacher verwendet werden. Sie zeichnen sich gegenüber bekannten Systemen für die Modifizierung von Kunststoffen wie PVC durch eine gute Lagerstabilität in Plastisolen aus. Die erfindungsgemäßen Weichmacher bzw. die Terephthalsäuredialkylester, die eine längste Kohlenstoffkette von zumindest 4 Kohlenstoffatomen aufweisen und eine Gesamtzahl an Kohlenstoffatomen pro Alkylrest von 5 aufweisen, können z. B. in Farben und Lacken, Dichtungsmassen, in Klebstoffen oder Klebstoffkomponenten oder in Kunststoffen oder Kunststoffkomponenten verwendet werden. Vorzugsweise werden die erfindungsgemäßen Weichmacher bzw. die genannten Terephthalsäuredialkylester als Weichmacher in Kunststoffen, wie z. B. PVC, Polyalkylmethacrylaten (PAMA) oder PVB verwendet. Bevorzugt werden sie beispielsweise in PVC- oder PAMA-Plastisolen verwendet. Besonders bevorzugt werden sie in Abmischungen mit sogenannten Primärweichmachern eingesetzt. Unter Primärweichmachern werden solche Weichmacher verstanden, die als alleinige Weichmacher eingesetzt werden können und in weiten Konzentrationsbereichen mit dem Polymer verträglich sind. Zu diesen Primärweichmachern werden beispielsweise Phthalsäuredialkylester, Terephthalsäuredialkylester und Trimellitsäuretrialkylester gerechnet, die jeweils in der Alkylseitenkette über 7 bis 11C-Atome verfügen. Weiterhin zählen die aus diesen genannten Produkten beispielsweise durch Kernhydrierung herstellbaren Cyclohexandicarbonsäureester oder Cyclohexantricarbonsäureester mit zu diesen Primärweichmachern. Als weitere Primärweichmacher seien hier genannt: Alkylsulfonsäureester des Phenols (z. B. Markenname MESAMOLL oder MESAMOLL II), Glycoldibenzoate, Glycerinester, acylierte oder nicht acylierte Citronensäuretrialkylester, Polymerweichmacher.

Durch die Verwendung der Terephthalsäuredialkylester, bei denen die Alkylreste des verwendeten Terephthalsäuredialkylesters eine längste Kohlenstoffkette von zumindest 4 Kohlenstoffatomen aufweisen und eine Gesamtzahl an Kohlenstoffatomen pro Alkylrest von 5 aufweisen, als Weichmacher sind entsprechende Zusammensetzungen erhältlich. Diese Zusammensetzungen enthaltend einen Weichmacher und ein Polymer, ausgewählt aus PVC, PVB, PAMA oder Homo- oder Copolymere auf Basis von Ethylen, Propylen, Butadien, Vinylacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, cyclischen Olefinen, zeichnen sich eben dadurch aus, dass sie als Weichmacher einen Terephthalsäuredialkylester enthalten, bei dem die Alkylreste eine längste Kohlenstoffkette von zumindest 4 Kohlenstoffatomen aufweisen und eine Gesamtzahl an Kohlenstoffatomen pro Alkylrest von 4 bis 5, insbesondere 4 oder 5, aufweisen.

Es kann vorteilhaft sein, wenn Terephthalsäuredialkylester als Weichmacher in der erfindungsgemäßen Zusammensetzung enthalten sind, bei denen die Alkylreste des Terephthalsäuredialkylesters eine Gesamtzahl an Kohlenstoffatomen pro Alkylrest von 4 aufweisen. Bevorzugt enthält eine solche Zusammensetzung einen Terephthalsäuredialkylester, dessen Alkylreste von 70 bis 100 % (Massen-% bezogen auf die Summe der Alkylreste) n-Butyl-Reste, vorzugsweise von 85 bis 99,9 % n-Butyl-Reste. Diese erfindungsgemäßen Weichmacher haben den Vorteil, dass sie gegenüber den Terephthalsäuredialkylester, bei denen die Alkylreste eine Gesamtzahl an Kohlenstoffatomen pro Alkylrest von 5 aufweisen, eine bessere Gelierung bewirken.

Besonders vorteilhaft kann es sein, wenn Terephthalsäuredialkylester als Weichmacher in der erfindungsgemäßen Zusammensetzung enthalten sind, bei denen die Alkylreste des Terephthalsäuredialkylesters eine Gesamtzahl an Kohlenstoffatomen pro Alkylrest von 5 aufweisen. Diese erfindungsgemäßen Weichmacher haben den Vorteil, dass sie gegenüber den Terephthalsäuredialkylestern, bei denen die Alkylreste eine Gesamtzahl an Kohlenstoffatomen pro Alkylrest von 4 aufweisen, eine niedrigere Flüchtigkeit aufweisen.

Vorzugsweise weist die erfindungsgemäße Zusammensetzung Terephthalsäuredialkylester auf, deren Alkylreste zu mehr als 60 % (Massen-% bezogen auf die Summe der Alkylreste) n-Pentyl-Reste sind. Unter Terephthalsäure-dialkylestern werden dabei auch Mischungen von Terephthalsäuredialkylestern verstanden, die Terephthalsäuredialkylester mit unterschiedlichen Alkylresten aufweisen. Bevorzugt weisen die erfindungsgemäßen Zusammensetzungen Terephthalsäuredialkylester auf, deren Alkylreste von 70 bis 99,9 % n-Pentyl-Reste und von 30 bis 0,1 % Methylbutyl-Reste, insbesondere 2-Methylbutylreste, besonders bevorzugt von 85 bis 98 % n-Pentyl-Reste und von 15 bis 2 % Methylbutyl-Reste, insbesondere 2-Methylbutylreste, und ganz besonders bevorzugt von 90 bis 96 % n-Pentyl-Reste und von 10 bis 4 % Methylbutyl-Reste, insbesondere 2-Methylbutylreste sind. Vorzugsweise sind die Methylbutyl-Reste zu mehr als 50 %, bevorzugt zu mehr als 75 % und besonders bevorzugt zu mehr als 95 % 2-Methylbutyl-Reste. Die vorgenannten Bereiche sind insbesondere dann bevorzugt, wenn erfindungsgemäß Terephthalsäuredialkylester verwendet werden, die eine Gesamtzahl an Kohlenstoffatomen pro Alkylrest von 5 aufweisen. Ganz besonders bevorzugt enthält die erfindungsgemäße Zusammensetzung einen Terephthalsäuredialkylester, dessen Alkylreste zu weniger als 0,5 Massen-%, (bezogen auf die Summe der Alkylreste) vorzugsweise zu weniger als 0,2 und besonders bevorzugt von 0,001 bis 0,1 Massen-% 3-Methylbutyl-Reste sind. Die prozentuale Verteilung der C₅-Alkyl-Reste kann auf einfache Weise durch Verseifen der Ester, Abtrennen des erhaltenen Alkohols und gaschromatographische (GC) Analyse des Alkohols ermittelt werden.

Es ist vorteilhaft, wenn die erfindungsgemäße Zusammensetzung neben Terephthalsäuredialkylester bei dem die Alkylreste eine längste Kohlenstoffkette von zumindest 4 Kohlenstoffatomen aufweisen und eine Gesamtzahl an Kohlenstoffatomen pro Alkylrest von 5 aufweisen, zumindest einen weiteren primären Weichmacher aufweist. Unter primären Weichmachern werden solche Verbindungen verstanden, die als alleinige Weichmacher eingesetzt werden können und in weiten Konzentrationsbereichen mit dem weichzumachenden Polymer verträglich sind. Durch das Vorhandensein von primären Weichmachern können die Eigenschaften der erfindungsgemäßen Zusammensetzung variiert werden. Vorzugsweise weist die erfindungsgemäße Zusammensetzung neben den genannten Terephthalsäuredialkylester zumindest einen weiteren primären Weichmacher, ausgewählt aus den Phthalsäuredialkylestern, Trimellisäuretrialkylestern, Adipinsäuredialkylestern, Terephthalsäuredialkylestern, 1,2-Cyclohexandisäurealkylestern, 1,3-Cyclohexandisäurealkyl-estern und 1,4-Cyclohexandisäurealkylestern mit Alkyl = Alkylrest mit 7 bis 11, insbesondere 8 bis 10 Kohlenstoffatomen, den Glycoldibenzoaten und den Alkylsulfonsäureestern von Phenol mit Alkyl = Alkylrest mit 8 bis 22 Kohlenstoffatomen sowie den acylierten und nicht acylierten Citronensäuretrialkylestern, Polymerweichmachern und Glycerinestern auf.

Der Anteil an primären Weichmachern beträgt in der erfindungsgemäßen Zusammensetzung vorzugsweise von 20 bis 99 Massen-% in Bezug auf die Summe der in der Zusammensetzung enthaltenen primären Weichmacher und Terephthalsäuredialkylester, bei denen die Alkylreste eine längste Kohlenstoffkette von zumindest 4 Kohlenstoffatomen aufweisen und eine Gesamtzahl an Kohlenstoffatomen pro Alkylrest von 5 aufweisen. Bevorzugt beträgt der Anteil der primären Weichmacher von 25 bis 80 Massen-% und besonders bevorzugt von 30 bis 75 Massen-%.

Es ist vorteilhaft, wenn die erfindungsgemäße Zusammensetzung neben Terephthalsäuredialkylestern, bei denen die Alkylreste eine längste Kohlenstoffkette von zumindest 4 Kohlenstoffatomen aufweisen und eine Gesamtzahl an Kohlenstoffatomen pro Alkylrest von 5 aufweisen, zumindest einen Benzoesäurealkylester mit Alkyl = Alkylrest mit 8 bis 10 Kohlenstoffatomen, vorzugsweise Benzoesäurenonyl- oder Benzoesäureisononylester und besonders bevorzugt Benzoesäureisononylester aufweist. Der Anteil an Benzoesäurealkylester mit Alkyl = Alkylrest mit 8 bis 10 Kohlenstoffatomen, insbesondere an Benzoesäureisononylester beträgt in der erfindungsgemäßen Zusammensetzung vorzugsweise von 5 bis 90 Massen-%, bevorzugt von 10 bis 80 Massen-% und besonders bevorzugt von 30 bis 70 Massen-% bezogen auf die Summe der in der Zusammensetzung enthaltenen Benzoesäurealkylester und Terephthalsäuredialkylester, bei denen die Alkylreste eine längste Kohlenstoffkette von zumindest 4 Kohlenstoffatomen aufweisen und eine Gesamtzahl an Kohlenstoffatomen pro Alkylrest von 4 bis 5 aufweisen.

Ganz besonders vorteilhaft ist es, wenn erfindungsgemäße Zusammensetzungen neben Terephthalsäuredialkylestern, bei denen die Alkylreste eine längste Kohlenstoffkette von zumindest 4 Kohlenstoffatomen aufweisen und eine Gesamtzahl an Kohlenstoffatomen pro Alkylrest von 5 aufweisen, und zumindest einem Benzoesäurealkylester mit Alkyl = Alkylrest mit 8 bis 10 Kohlenstoffatomen, zumindest einen primären Weichmacher aufweisen. Solche erfindungsgemäßen Zusammensetzungen weisen vorzugsweise einen Anteil an Terephthalsäuredialkylestern mit einer Gesamtzahl an Kohlenstoffatomen von 4 bis 5 pro Alkylrest von 5 bis 70 Massen-%, einen Anteil an Benzoesäurealkylester mit Alkyl = Alkylrest mit 8 bis 10 Kohlenstoffatomen von 5 bis 70 Massen-% und einen Anteil an primären Weichmachern von 20 bis 90 Massen-% auf.

Neben den genannten Bestandteilen kann die erfindungsgemäße Zusammensetzung weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl, Gleitmittel, Treibmittel, Kicker, Antioxidanzien oder Biozide. Als weitere Weichmacher können die erfindungsgemäßen Zusammensetzungen insbesondere Ester von Cyclohexandicarbonsäure, Phthalsäure oder Adipinsäure aufweisen.

Als Vertreter der oben genannten Polymere kann die erfindungsgemäße Zusammensetzung insbesondere Polyacrylate mit gleichen oder verschiedenen Alkylresten mit 4 bis 10 C-Atomen, gebunden am Sauerstoffatom der Estergruppe, insbesondere mit dem n-Butyl-, n-Hexyl-, n-Octyl-, Isononyl- und 2-Ethylhexylrest, Polymethacrylat, Polymethylmethacrylat, Methylacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose aufweisen.

Die erfindungsgemäßen Zusammensetzungen können als Polymer insbesondere PVC aufweisen. Vorzugsweise enthält die erfindungsgemäße Zusammensetzung als PVC-Typ Suspensions-, Masse-, Mikrosuspensions- oder Emulsions-PVC.

Die erfindungsgemäße Zusammensetzung kann insbesondere ein Plastisol, ein Kunstleder, ein Fußbodenbelag, ein Unterbodenschutz, ein beschichtetes Gewebe, eine Dichtmasse, eine Tapete, ein Lack, eine Farbe, eine Tinte oder ein Klebstoff sein oder zur Herstellung dieser Produkte verwendet werden.

Bevorzugt werden die erfindungsgemäßen Zusammensetzungen zur Herstellung von Plastisolen, insbesondere von denen des PVC, mit besonders vorteilhaften verarbeitungstechnischen Eigenschaften eingesetzt. Diese Plastisole können in zahlreichen Produkten wie beispielweise Kunstleder, Fußböden, Tapeten etc. bzw. zu deren Herstellung eingesetzt werden. Unter diesen Anwendungen besonders bevorzugt ist die Verwendung in cushion vinyl (CV)-Fußböden, hier insbesondere in der Deckschicht, wo eine weitere Verbesserung in der Fleckbeständigkeit ("Stain Resistance") bewirkt wird. Durch Verwendung der erfindungsgemäßen Gemische als Rezepturbestandteil können Plastisole mit niedriger Viskosität sowie erhöhter Lagerstabilität und gleichzeitig mit beschleunigter Gelierung und verbesserter Kälteflexibilisierung erhalten werden. Weiterhin bevorzugt ist auch die Verwendung in chemisch oder mechanisch geschäumten Schichten oder in Kompaktschichten bzw. Grundierungen.

Die erfindungsgemäß eingesetzten Terephthalsäureester können aus den entsprechenden Alkoholen oder Alkoholgemischen durch Umsetzung mit Terephthalsäure oder deren Derivaten hergestellt werden. Insbesondere können die erfindungsgemäß eingesetzten Terephthalsäureester entweder durch Veresterung mit Terephthalsäure oder bevorzugt durch Umesterung aus Terephthalsäureestern mit kürzeren Alkoholresten hergestellt werden. Im Falle der Umesterung ist als Ausgangsstoff Dimethylterephthalat, ein industrielles Massenprodukt, besonders bevorzugt.

Alkohole zur Herstellung der erfindungsgemäßen Terephthalsäuredialkylester können alle Alkohole sein, deren längste Kohlenstoffkette aus 5 C-Atomen besteht. Hierbei sind primäre Alkohole bevorzugt. Beispielhaft sei hier n-Pentanol genannt.

Bevorzugt können zur Herstellung der erfindungsgemäßen Terephthalsäuredialkylester primäre Alkohole oder Alkoholgemische wie sie beispielsweise durch Hydroformylierung eines Alkens mit nachfolgender Hydrierung erhalten werden können, eingesetzt werden. So kann zum Beispiel n-Butanol durch Hydroformylierung von Propylen und nachfolgender Hydrierung des Butyraldehyds zu n-Butanol hergestellt werden. Der bei der Hydroformylierung von Propylen ebenfalls entstehende iso-Butyraldehyd kann optional destillativ abgetrennt werden.

Vorstufen für Pentanole sind vorzugsweise technische Kohlenwasserstoffgemische, die ein oder mehrere Olefin(e) mit 4 Kohlenstoffatomen enthalten. Die bedeutendste Quelle für C₄-Olefine ist der C₄-Schnitt des Crackbenzins aus Steamcrackern. Daraus wird nach Extraktion(sdestillation) des Butadiens oder dessen Selektivhydrierung zu einem n-Butengemisch ein Kohlenwasserstoffgemisch (Raffinat I oder hydriertes Crack-C₄) hergestellt, das Isobuten, 1-Buten und die beiden 2-Butene enthält. Ein anderer Rohstoff für C₄-Olefine ist der C₄-Schnitt aus FCC-Anlagen, der wie oben beschrieben aufgearbeitet werden kann. C₄-Olefine, hergestellt durch Fischer-Tropsch-Synthese, sind nach Selektivhydrierung des darin enthaltenen Butadiens zu n-Butenen ebenfalls ein geeigneter Einsatzstoff. Darüber hinaus können Olefingemische, die durch Dehydrierung von C₄-Kohlenwasserstoffen oder durch Metathesereaktionen erhalten werden, oder andere technische Olefinströme geeignete Einsatzstoffe sein. Neben dem Raffinat I sind als Vorstufen für die Pentanole auch Raffinat II, Raffinat III, ein Strom welcher durch Abtrennung des größten Teils an 1-Buten aus dem Raffinat II erhalten wird, und sogenanntes Rohbutan, welches nach einer Oligomerisierung von Raffinat II anfällt und welches neben Alkanen als Olefin ausschließlich geringe Mengen von 2-Buten aufweist, geeignet. Der Vorteil der Verwendung von Raffinat II, Raffinat III oder Rohbutan als Vorstufe für Pentanole liegt darin, dass diese Vorstufen kein bzw. nahezu kein Isobuten aufweisen, weshalb die erhaltenen Pentanole kein bzw. nur geringe Mengen (kleiner 0,5 Massen-% in Bezug auf die Pentanole) an 3-Methylbutanol aufweisen.

Wegen des häufig sehr hohen Trennaufwands zur Trennung der Einsatzgemische kann es vorteilhaft sein, die im als Einsatzgemisch einzusetzenden technischen Gemisch vorliegenden Olefine nicht zu trennen, sondern die Gemische direkt einzusetzen.

Besonders bevorzugt werden die erfindungsgemäß eingesetzten Terephthalsäuredialkylester hergestellt durch ein Verfahren zur Herstellung von Terephthalsäuredialkylestern, deren Alkylreste eine längste Kohlenstoffkette von zumindest 4 Kohlenstoffatomen aufweisen und eine Gesamtzahl an Kohlenstoffatomen pro Alkylrest von 5 aufweisen, umfassend die Schritte,
a) Hydroformylierung eines C₄-Olefins unter Erhalt eines C₅- Aldehyds,
b) Hydrierung des in Schritt a) erhaltenen Aldehyds zum entsprechenden Alkohol und
c) Umsetzung des Alkohols aus b) mit Terephthalsäure oder einem Derivat der Terephthalsäure zum entsprechenden Diester.

Es kann vorteilhaft sein, wenn nach Verfahrensschritt a) (Hydroformylierung) und/oder b) (Hydrierung) eine Auftrennung der in diesen Verfahrensstufen erhaltenen Produktgemische in die einzelnen Isomeren erfolgt. Eine solche Auftrennung kann z. B. thermisch, insbesondere durch Destillation erfolgen.

### Verfahrensschritt a)

Die Hydroformylierung aller Olefine im Einsatzgemisch kann in einer Stufe erfolgen. Dies kann insbesondere dann vorteilhaft sein, wenn bei der Hydroformylierung von Propen nur eine olefinische Verbindung im Einsatzgemisch vorhanden ist. Die Hydroformylierung von ausschließlich Propen als Olefin enthaltenden Einsatzgemischen kann in einer Stufe unter den unten für die erste Stufe beschriebenen Bedingungen und mit dem dort beschriebenen Katalysator durchgeführt werden.

Sollen Pentanole hergestellt werden, können in Verfahrenschritt a) ebenfalls isomerenreine Butene, wie z. B. 1-Buten, 2-Buten und Isobuten eingesetzt werden. Da die isomerenreinen Olefine als Einsatzgemische aber häufig nicht vorliegen, sondern meistens technische Gemische von C₄-Kohlenwasserstoffen, wie sie oben beschrieben wurden, als Einsatzgemische vorliegen, wird im erfindungsgemäßen Verfahren in Verfahrensschritt a) vorzugsweise ein Gemisch von Olefinen eingesetzt, welches Isobuten und/oder 1-Buten und 2-Butene aufweist.

Die Hydroformylierung der im Einsatzgemisch enthaltenen Olefine kann wiederum in einer Stufe erfolgen. Dazu wird vorzugsweise ein Katalysator eingesetzt, der vermag, Olefine mit unterschiedlicher Lage der Doppelbindung und/oder Anzahl der Verzweigungen zu hydroformylieren. Katalysatoren, die dafür geeignet sind, bewirken aber meistens nur eine geringe Selektivität für die Bildung von Produkten (Aldehyde, Alkohole, Formiate), die durch endständige Hydroformylierung entstanden sind, und/oder zeigen eine zu geringe Reaktionsgeschwindigkeit für ein technisches Verfahren.

Wenn aus dem Hydroformylierungsprodukten Einsatzalkohole, insbesondere Pentanole bzw. Pentanolgemische mit möglichst geringem Verzweigungsgrad gewonnen werden sollen, ist es zweckmäßig, die Hydroformylierung derart durchzuführen, dass ein hoher Anteil an Produkten, die durch endständige Hydroformylierung entstanden sind, gewonnen wird; denn nur die endständig hydroformylierten Produkte weisen den gleichen Verzweigungsgrad wie ihre Ausgangsolefine auf, bei mittelständiger Hydroformylierung dagegen erhöht sich der Verzweigungsgrad des entstandenen Produkts um 1.

Die in einem technischen Gemisch vorliegenden Olefine unterscheiden sich in ihrer Reaktivität bei der Hydroformylierung beträchtlich. Im Allgemeinen sind Olefine mit endständigen Doppelbindungen reaktiver als Olefine mit innenständigen Doppelbindungen und lineare Olefine reaktiver als verzweigte. Speziell im Falle der C₄-Olefine gilt, 1-Buten ist reaktiver als Isobuten und Isobuten ist reaktiver als die beiden 2-Butene (cis bzw. trans). Diese unterschiedliche Reaktivität kann genutzt werden, um einen hohen Anteil an Produkten, die durch endständige Hydroformylierung entstanden sind, zu gewinnen, d. h., aus 1-Buten soll hauptsächlich Valeraldehyd und nicht 2-Methylbutanal, aus Isobuten 3-Methylbutanal und nicht 2,2-Dimethylpropanal sowie aus den beiden 2-Butenen möglichst viel Valeraldehyd (n-Pentanal) und wenig 2-Methylbutanal entstehen.

Da es bislang keinen Katalysator gibt, der simultan mit zufriedenstellender Geschwindigkeit sowohl die Umsetzung von 1-Buten als auch von Isobuten und den 2-Butenen zu Produkten, die durch endständige Hydroformylierung entstanden sind, bewirkt, wird die Hydroformylierung, insbesondere wenn die Einsatzgemische sowohl Isobuten und/oder 1-Buten als auch 2-Butene enthalten, vorzugsweise zumindest zweistufig durchgeführt. Wird das erfindungsgemäße Verfahren zweistufig durchgeführt, wird vorzugsweise in einer Stufe Isobuten und/oder 1-Buten und in der anderen Stufe 2-Butene hydroformyliert.

In einer ersten Stufe wird die Hydroformylierung vorzugsweise mit einem geeigneten Katalysator unter Bedingungen vorgenommen, bei denen nur α-Olefine (1-Buten, Isobuten), jedoch nicht die 2-Butene zu den entsprechenden Aldehyden umgesetzt werden. Dabei werden die Bedingungen vorzugsweise derart gewählt, dass 1-Buten möglichst selektiv zu Valeraldehyd und Isobuten möglichst selektiv zu 3-Methylbutanal umgesetzt wird. Als Katalysatoren können beispielsweise Verbindungen eingesetzt werden, die Rhodium und triorganische Phosphorverbindungen, insbesondere Phosphine als Liganden aufweisen. Die Umsetzung kann in homogener Phase (analog dem UCC-Verfahren, beschrieben in EP 0 562 451) oder in heterogener Phase (analog dem Rhone-Poulenc-Ruhrchemie-Verfahren, beschrieben in DE 026 27 354 und EP 0 562 451) durchgeführt werden. Wegen der leichteren Katalysatorabtrennung wird die erste Stufe des Verfahrensschrittes a) bevorzugt gemäß dem zweiten Verfahren durchgeführt. Die Reaktionstemperaturen für die erste Stufe des Verfahrensschrittes a) betragen bevorzugt von 70 bis 150 °C, vorzugsweise von 100 bis 130 °C. Die Verfahrensdrücke betragen vorzugsweise von 2 bis 20 MPa, bevorzugt von 3 bis 6 MPa.

Optional kann die Hydroformylierung der 1-Olefine im Mehrphasensystem, wobei Edukt, Produkt und Synthesegas in einer kontinuierlichen Katalysatorphase dispergiert sind, unter hohen Leerrohrgeschwindigkeiten durchgeführt werden. Solche Verfahren werden beispielsweise in DE 199 25 384 A1 und DE 199 57528 A1 beschrieben, auf welche hier ausdrücklich verwiesen wird.

Die Hydroformylierung der 1-Olefine in der ersten Stufe des Verfahrensschrittes a) kann einstufig oder zweistufig durchgeführt werden. Bei der zweistufigen Hydroformylierung wird im ersten Reaktor vorwiegend 1-Buten und im zweiten Reaktor hauptsächlich Isobuten umgesetzt. In beiden Reaktoren können die gleichen Katalysatoren oder unterschiedliche Katalysatoren eingesetzt werden. Bei Verwendung gleicher Katalysatoren ist eine gemeinsame Katalysatoraufarbeitung möglich.

Nach der gerade beschriebenen Hydroformylierung von 1-Buten und Teilen des Isobutens in der ersten Stufe des Verfahrensschrittes a) verbleiben im Einsatz-Kohlenwasserstoffgemisch falls vorhanden, die 2-Butene und gegebenenfalls Isobuten und höchstens Spuren von 1-Buten. Dieses Gemisch kann als solches unter Verwendung eines anderen Katalysatorsystems oder nach Auftrennung in zwei Fraktionen, von denen eine Isobuten und die andere die beiden 2-Butene enthält, hydroformyliert werden. Bevorzugt wird das Gemisch aufgetrennt und die Isobuten aufweisende Fraktion und die 2-Butene aufweisende Fraktion werden getrennt hydroformyliert.

Das Isobuten bzw. die Isobuten aufweisende Fraktion kann in hohen Selektivitäten zu 3-Methylbutanal hydroformyliert werden. Geeignete Katalysatoren dafür sind Rhodiumkomplexe, die ein- oder mehrzähnige Phosphitliganden enthalten. Geeignete einzähnige Phosphitliganden sind beispielsweise Triarylphosphite, deren Arylgruppen sowohl in ortho-Stellung zum Phosphit-Sauerstoff eine sperrige Gruppe aufweisen als auch in m-oder p-Stellung substituiert sind, wie z. B. Tris(2,4-di-tert.-butyl-phenyl)phosphit Die Hydroformylierung von Isobuten unter Verwendung eines Katalysatorsystems, das aus Rhodium und einem Bisphosphit besteht, wird beispielsweise in den Patentschriften US 4,668,651, US 4,769,498 und WO 85/03702 beschrieben, auf welche ausdrücklich verwiesen wird und deren Offenbarungsgehalt Gegenstand der vorliegenden Beschreibung ist.

Optional kann die abgetrennte Isobutenfraktion ganz oder teilweise in die vorgeschaltete erste Hydroformylierungsstufe zurückgeführt werden. Dabei kann es besonders vorteilhaft sein, vom Isobuten die gesättigten Kohlenwasserstoffe abzutrennen, was z. B. thermisch erfolgen kann. Nach einer solchen Abtrennung der gesättigten Kohlenwasserstoffe kann es besonders vorteilhaft sein, das Isobuten vollständig in die vorgeschaltete erste Hydroformylierungsstufe zurückzuführen.

Die Hydroformylierung von 2-Butenen bzw. 2-Butenen aufweisenden Fraktionen kann mit Hilfe von verschiedenen bekannten Katalysatoren durchgeführt werden, wobei üblicherweise ein Gemisch aus 2-Methylbutanal und Valeraldehyd entsteht. In den meisten Fällen ist 2-Methylbutanal das Hauptprodukt. Die Verwendung von unmodifizierten Kobaltkatalysatoren als Katalysator für die Hydroformylierung von 2-Butenen wird in EP 0 646 563 und die Verwendung von unmodifiziertem Rhodium wird in EP 0 562 451 beschrieben. Weiterhin kann für die Hydroformylierung von 2-Butenen das gleiche Katalysatorsystem, das für die Hydroformylierung von Isobuten eingesetzt wird, nämlich ein Komplex aus Rhodium und einzähniges Triarylphosphit verwendet werden. Hohe Selektivitäten an Valeraldehyd können bei der Verwendung eines Katalysators, bestehend aus Rhodium und sperrigen aromatischen Bisphosphiten, erhalten werden, wie sie beispielsweise in EP 0 213 639, EP 0 214 622 oder US 5,763,680 beschrieben werden. Allerdings sind die Reaktionsgeschwindigkeiten für ein technisches Verfahren relativ gering. Besonders bevorzugt wird als Bisphosphit-Ligand der in US 5,763,680 als Ligand D bezeichnete Ligand eingesetzt.

Wie oben ausgeführt ist, können die im Einsatzstoff vorliegenden Olefine getrennt oder gemeinsam hydroformyliert werden. Wenn der Linearität der Endprodukte keine große Bedeutung zukommt, ist es zweckmäßig, die Olefine gemeinsam zu hydroformylieren. Ist dagegen ein möglichst wenig verzweigtes Endprodukt erwünscht, ist es bevorzugt, die Hydroformylierung in mindestens zwei Stufen durchzuführen. Im Falle eines C₄-Olefingemisches bedeutet der letztere Fall, dass im ersten Reaktor 1-Buten und gegebenenfalls Isobuten umgesetzt wird und in dem/den nachgeschalteten Reaktor(en) optional die restlichen Olefine.

Aus den Hydroformylierungsgemischen kann der Katalysator nach bekannten Verfahren abgetrennt werden. Beispielsweise kann bei Verfahren, bei denen der Rhodium-Katalysator homogen im Reaktionsgemisch vorliegt, der Katalysator destillativ abgetrennt werden. Bei der Umsetzung in heterogener Phase (zwei flüssige Phasen) kann die Abtrennung des Katalysators z. B. durch Phasentrennung erfolgen (Ed. B. Cornils, W. A. Herrmann, Applied Homogeneous Catalysis with Organic Compounds, Vol. 1, S. 80, VCH-Verlag, 1996).

### Verfahrensschritt b)

Die Hydroformylierungsgemische können nach der Entkatalysierung entweder direkt in der Hydrierung eingesetzt werden oder aber zuvor destillativ oder mit anderen Trennmethoden in zwei oder mehrere Fraktionen aufgetrennt werden. Insbesondere kann es vorteilhaft sein, das Hydroformylierungsgemisch so aufzuarbeiten, dass eine oder mehrere im Wesentlichen Aldehyde aufweisende Fraktionen erhalten werden.

Die entkatalysierten Hydroformylierungsgemische oder die daraus durch ein Trennverfahren wie z. B. Destillation abgetrennten Aldehyde oder Aldehyd aufweisenden Fraktionen werden erfindungsgemäß hydriert. Dabei können die Hydroformylierungsgemische getrennt oder gemeinsam hydriert werden. Durch die Hydrierung entstehen aus den Aldehyden die entsprechenden gesättigten Alkohole. Dies sind beispielsweise Butanole, n-Pentanol, 2-Methylbutanol und 3-Methylbutanol.

Zur Hydrierung können z. B. Nickel-, Kupfer-, Kupfer/Nickel-, Kupfer/Chrom-, Kupfer/Chrom/Nickel-, Zink/Chrom-, Nickel/Molybdän-Katalysatoren verwenden werden. Die Katalysatoren können trägerfrei sein, oder die hydrieraktiven Stoffe bzw. ihre Vorläufer können auf Trägern, wie beispielsweise Siliziumdioxid oder Aluminiumdioxid, aufgebracht sein. Bevorzugte Katalysatoren, die in Verfahrensschritt b) eingesetzt werden und an denen die Hydroformylierungsgemische hydriert werden können, weisen jeweils 0,3 bis 15 Massen-% Kupfer und Nickel sowie als Aktivatoren 0,05 bis 3,5 Massen-% Chrom und optional 0,01 bis 1,6 Massen-%, vorzugsweise 0,02 bis 1,2 Massen-% einer Alkalikomponente auf einem Trägermaterial, vorzugsweise Aluminiumoxid und Siliziumdioxid auf. Die Mengenangaben beziehen sich auf den noch nicht reduzierten Katalysator. Die Alkalikomponente ist optional. Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudaten oder Ringen. Sie werden zweckmäßig vor ihrem Einsatz aktiviert, z. B. durch Erhitzen im Wasserstoffstrom.

Die Hydrierung, kann eine Gasphasen- oder Flüssigphasenhydrierung sein. Bevorzugt wird die Hydrierung bei einem Gesamtdruck von 0,5 bis 50 MPa, vorzugsweise von 1,5 bis 10 MPa durchgeführt. Eine Hydrierung in der Gasphase kann auch bei niedrigeren Drücken durchgeführt werden, wobei dann entsprechend große Gasvolumina vorliegen. Werden mehrere Hydrierungsreaktoren eingesetzt, können die Gesamtdrücke in den einzelnen Reaktoren innerhalb der genannten Druckgrenzen gleich oder verschieden sein. Die Reaktionstemperaturen können bei der Hydrierung in flüssiger oder gasförmiger Phase in der Regel von 120 bis 220 °C, insbesondere von 140 bis 180 °C betragen. Solche Hydrierungen sind beispielsweise in den Patentanmeldungen DE 198 42 369 und DE 198 42 370 beschrieben, auf welche hier ausdrücklich verwiesen wird.

Die Hydrierung wird bevorzugt in Gegenwart von Wasser durchgeführt. Das benötigte Wasser kann im Reaktorzulauf enthalten sein. Es ist jedoch auch möglich, Wasser an geeigneter Stelle in die Hydrierapparatur einzuspeisen. Bei Gasphasenhydrierung wird Wasser zweckmäßig in Form von Wasserdampf zugeführt. Ein bevorzugtes Hydrierverfahren ist die Flüssigphasenhydrierung unter Zusatz von Wasser, wie sie beispielsweise in DE 100 62 448 beschrieben ist. Besonders bevorzugt wird die Hydrierung bei einem Wassergehalt von 0,05 bis 10 Massen-%, insbesondere 0,5 bis 5 Massen-%, ganz besonders 1 bis 2,5 Massen-% durchgeführt. Der Wassergehalt wird dabei im Hydrieraustrag bestimmt.

Die aus der Hydrierung erhaltenen Gemische können entweder direkt in der Veresterungsstufe bzw. Umesterungsstufe eingesetzt werden oder aber destillativ oder mit anderen Trennmethoden in zwei oder mehrere Fraktionen aufgetrennt werden. Insbesondere kann es vorteilhaft sein, das Hydrierungsgemisch so aufzuarbeiten, dass eine oder mehrere Fraktionen von Alkoholen mit der gleichen Anzahl an Kohlenstoffatomen erhalten werden.

Bevorzugt kann die destillative Aufarbeitung so durchgeführt werden, dass es zu einer weitgehenden Auftrennung in die einzelnen Bestandteile kommt.

Wenn ein Terephthalsäureester von linearen Alkoholen als Einsatzalkoholen hergestellt werden soll, können die linearen Alkohole, wie z. B. n-Pentanol von den verzweigten Alkoholen, wie z. B. verzweigte Pentanole destillativ abgetrennt werden.

### Verfahrensschritt c)

Die Umsetzung der in Verfahrensschritt b) erhaltenen Einsatzalkohole zum entsprechenden Diester kann durch Reaktion mit Terephthalsäure oder einem Derivat der Terephthalsäure, insbesondere einem Terephthalsäureester, erfolgen. Bevorzugt wird in Verfahrensschritt c) eine Veresterung von Terephthalsäure oder eine Umesterung von Dimethylterephthalat mit dem aus Schritt b) erhaltenen Alkohol durchgeführt.

Die erfindungsgemäßen Terephthalsäureester können z. B. durch Veresterung von Terephthalsäure mit den entsprechenden Alkoholen erhalten werden. Der zur Bildung des Esters eingesetzte Alkohol bzw. das Alkoholgemisch, das gleichzeitig als Schleppmittel zur Abtrennung des bei der Reaktion entstehenden Wassers dienen kann, wird vorzugsweise im Überschuss, bevorzugt mit einem Überschuss von 5 bis 50 %, insbesondere 10 bis 30 % der zur Bildung des Esters notwendigen molaren Menge eingesetzt.

Bei der Veresterung mit Butanolen oder Pentanolen oder Butanol- oder Pentanolgemischen ist bedingt durch den hohen Schmelzpunkt der Terephthalsäure und der vergleichsweise schlechten Löslichkeit der Terephthalsäure in diesen Alkoholen die Reaktion unter Normaldruck relativ langsam, da die Temperatur nur geringfügig über den Siedepunkt der Alkohole bzw. des Alkoholgemisches angehoben werden kann. Zur Erhöhung der Veresterungsgeschwindigkeit kann es deshalb vorteilhaft sein, die Reaktion bei erhöhtem Druck und damit auch einer erhöhten Reaktionstemperatur durchzuführen. Optional kann die Reaktion auch mit einem anfänglichen Alkoholunterschuss durchgeführt werden. Hierbei wird erst im Verlaufe der Reaktion langsam die vollständige Alkoholmenge (gegebenenfalls inklusive dem bevorzugten Überschuss) zugegeben.

Als Veresterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder in Form von löslichen organischen Verbindungen verwendet werden können. Die Metallkatalysatoren sind im Vergleich zu den Katalysatoren auf Basis von Protonensäuren Hochtemperaturkatalysatoren, die ihre volle Aktivität oft erst bei Temperaturen oberhalb 180 °C erreichen. Es kann vorteilhaft sein, solche Metallkatalysatoren auf Basis von Metallen oder deren Verbindungen einzusetzen, da festgestellt wurde, dass mit diesen Katalysatoren im Vergleich zu Katalysatoren auf Basis von Protonensäuren weniger Nebenprodukte, wie beispielsweise Olefine aus dem eingesetzten Alkohol gebildet werden. Beispielhafte Vertreter für besonders bevorzugt eingesetzte Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat.

Die Katalysatorkonzentration kann in weiten Bereichen variiert werden und kann insbesondere abhängig von der Art des Katalysators variiert werden. Bei den bevorzugt eingesetzten Titanverbindungen beträgt die Katalysatorkonzentration vorzugsweise 0,005 bis 1,0 Massen-% bezogen auf das Reaktionsgemisch, bevorzugt 0,01 bis 0,5 Massen-% und besonders bevorzugt 0,01 bis 0,1 Massen-%.

Die Reaktionstemperaturen betragen bei der Verwendung von Katalysatoren auf Basis von Titan oder Titanverbindungen vorzugsweise von 160 °C bis 270 °C, bevorzugt von 180 bis 250 °C.

Im Allgemeinen hängen die optimalen Temperaturen für die Durchführung der Veresterung von den Einsatzstoffen, von dem Reaktionsfortschritt und von der Katalysatorkonzentration ab. Die optimalen Temperaturen können für jeden Einzelfall durch einfache Vorversuche leicht ermittelt werden. Durch die Verwendung von höheren Temperaturen kann die Reaktionsgeschwindigkeit erhöht werden allerdings werden Nebenreaktionen begünstigt, wie beispielsweise Wasserabspaltung aus Alkoholen oder Bildung farbiger Nebenprodukte.

Die gewünschte Reaktionstemperatur bzw. der gewünschte Temperaturbereich kann durch Anpassung des Drucks im Reaktionsgefäß eingestellt werden. Bei niedrig siedenden Alkoholen wird die Umsetzung deshalb vorzugsweise bei Überdruck und bei höher siedenden Alkoholen vorzugsweise bei vermindertem Druck durchgeführt. Im vorliegenden Fall wird die Veresterung deshalb vorzugsweise bei einem Überdruck, vorzugsweise bei einem Überdruck von 2 bis 8 bara durchgeführt.

Zur Entfernung des Reaktionswassers kann es vorteilhaft sein, wenn das Wasser als azeotropes Gemisch mit dem Alkohol aus dem Reaktionsgemisch abdestilliert wird. Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.

Die Rohestergemische, die neben dem/den Ester(n) Alkohol, Katalysator oder dessen Folgeprodukten und gegebenenfalls Nebenprodukte enthalten, können nach an sich bekannten Verfahren aufgearbeitet werden. Die Aufarbeitung umfasst dabei vorzugsweise folgende Schritte: Abtrennung des überschüssigen Alkohols und gegebenenfalls Leichtsieder, Neutralisation der vorhandenen Säuren, optional eine Wasserdampfdestillation, Umwandlung des Katalysators in einen leichtfiltrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann der gewünschte Ester, Dialkylester oder das Gemisch der Ester aus dem Reaktionsgemisch, gegebenenfalls nach Neutralisation des Ansatzes, destillativ ab- bzw. aufgetrennt werden. Dies kann insbesondere im Fall von Produkten, die bei Raumtemperatur fest sind, wie z. B. Di-3-methylbutyl-terephthalat, vorteilhaft sein.

Die erfindungsgemäßen Terephthalsäureester können in einer anderen Ausführungsform des erfindungsgemäßen Verfahrens durch Umesterung eines Terephthalsäuredialkylesters mit einem Einsatzalkohol, ausgewählt aus Butanol, Pentanol oder einem geeigneten Pentanol-Isomerengemisch gewonnen werden. Als Edukte werden Terephthalsäuredialkylester eingesetzt, deren am O-Atom der Estergruppe gebundenen Alkylreste 1 bis 3C-Atome aufweisen. Diese Reste können aliphatisch, geradkettig oder verzweigt, alicyclisch oder aromatisch sein. Eine oder mehrere Methylengruppen dieser Alkyl-Reste können durch Sauerstoff substituiert sein. Es kann vorteilhaft sein, wenn die dem Eduktester zugrunde liegenden Alkohole niedriger sieden als die Einsatzalkohole. Ein bevorzugter Einsatzstoff ist Dimethylterephthalat (DMT), der industriell hergestellt wird und deshalb in großen Mengen zur Verfügung steht.

Die Umesterung kann z. B. katalytisch, beispielsweise unter Verwendung von Brönstedt- oder Lewissäuren oder -basen als Katalysator durchgeführt werden. Unabhängig davon welcher Katalysator eingesetzt wird entsteht immer ein temperaturabhängiges Gleichgewicht zwischen den Einsatzstoffen (Dialkylterephthalat und Einsatzalkoholen) und den Produkten (erfindungsgemäßes Terephthalsäureester und freigesetzter Alkohol aus dem Einsatzdialkylterephthalat). Um das Gleichgewicht zu Gunsten des erfindungsgemäßen Terephthalsäureesters zu verschieben, kann es vorteilhaft sein, den aus dem Eduktester entstehende Alkohol aus dem Reaktionsgemisch abzudestillieren.

Auch bei dieser Ausführungsform des erfindungsgemäßen Verfahrens kann es vorteilhaft sein, den Alkohol insgesamt im Überschuss einzusetzen. Vorzugsweise wird der Einsatzalkohol in einem Überschuss von 5 bis 50 %, insbesondere 10 bis 30 % der zur Bildung des erfindungsgemäßen Terephthalsäuredialkylesters notwendigen molaren Menge eingesetzt.

Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet Metalle oder deren Verbindungen sind z. B. Zinn, Titan, Zirkonium, die als feinverteilte Metalle oder zweckmäßig in Form ihrer Salze, als Oxide oder in Form von löslichen organischen Verbindungen verwendet werden. Die Metallkatalysatoren sind im Vergleich zu den Katalysatoren auf Basis von Protonensäuren Hochtemperaturkatalysatoren, die ihre volle Aktivität oft erst bei Temperaturen oberhalb 180 °C erreichen. Es kann vorteilhaft sein, solche Metallkatalysatoren auf Basis von Metallen oder deren Verbindungen einzusetzen, da festgestellt wurde, dass mit diesen Katalysatoren im Vergleich zu Katalysatoren auf Basis von Protonensäuren weniger Nebenprodukte, wie beispielsweise Olefine aus dem eingesetzten Alkohol gebildet werden. Beispielhafte Vertreter für besonders bevorzugt eingesetzte Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat. Weiterhin können basische Katalysatoren, wie beispielsweise Oxide, Hydroxide, Hydrogencarbonate, Carbonate oder Alkoholate von Alkali- oder Erdalkalimetallen eingesetzt werden. Aus dieser Gruppe werden bevorzugt Alkoholate, wie beispielsweise Natriummethylat eingesetzt. Alkoholate können auch in situ aus einem Alkalimetall und einem Nonanol bzw. einem Isononanolgemisch hergestellt werden. Besonders bevorzugt werden solche Alkoholate eingesetzt, deren Alkoholrest mit einem der an der Reaktion beteiligten Alkohole übereinstimmt.

Die Katalysatorkonzentration kann in weiten Bereichen variiert werden und kann insbesondere abhängig von der Art des Katalysators variiert werden. Die Katalysatorkonzentration beträgt vorzugsweise von 0,005 bis 1,0 Massen-% bezogen auf das Reaktionsgemisch. Die für jeden Katalysator optimalen Konzentrationen können durch Vorversuche leicht bestimmt werden und ergeben sich aus einem Kompromiss aus möglichst geringem Katalysatorverbrauch (d. h. Kosten) und möglichst hoher Reaktionsgeschwindigkeit. Im Falle des besonders bevorzugten Tetrabutylorthotitanat liegt die bevorzugte Konzentration beispielsweise im Bereich von 0,05 bis 1 Massen-%, bezogen auf das eingesetzte Dialkylterephthalat.

Die Umesterung wird vorzugsweise bei einer Temperatur von 100 bis 220 °C durchgeführt. Die Temperatur wird besonders bevorzugt so hoch gewählt, dass der aus dem Eduktester entstehende Alkohol bei dem vorgegebenen Druck, vorzugsweise erhöhtem Druck, aus dem Reaktionsgemisch abdestilliert werden kann.

Aufgrund der niedrigen Siedepunkte der für die Umesterung einzusetzenden Alkohole (Butanole oder Pentanole) kann es zum Erreichen einer für die Katalyse mit Metallkatalysatoren wie Tetrabutylorthotitanat ausreichenden Temperatur vorteilhaft sein, die Reaktion entweder unter erhöhtem Druck oder zunächst mit einem deutlich unterstöchiometrischen Anteil an Einsatzalkohol durchzuführen. Unterhalb dieser Temperatur verläuft ansonsten die Umesterung mit nur sehr mäßiger Geschwindigkeit.

Wird die Reaktion zu Beginn der Reaktion mit einem unterstöchiometrischen Anteil an Einsatzalkohol durchgeführt, so hat es sich als vorteilhaft erwiesen, wenn nach Erreichen der Solltemperatur der restliche Alkohol so langsam zugegeben wird, dass die Temperatur des Reaktionsgemisches, gemessen im Sumpf, nicht unter diese Temperatur sinkt. Die bevorzugte Solltemperatur beträgt bei der Umesterung von DMT mit Butanol oder Pentanolen oder Pentanolgemischen mindestens 160 °C, bevorzugt mehr als 180 °C.

Die Aufarbeitung der Umesterungsgemische kann genauso wie für die Veresterungsgemische beschrieben erfolgen.

Die folgenden Beispiele sollen die Erfindung erläutern, ohne die Anwendungsbreite einzuschränken, die sich aus der Beschreibung und den Patentansprüchen ergibt.

### Beispiel 1: Herstellung von Di-n-Pentylterephthalat

In einen 2-Liter-Mehrhalskolben mit Destillationsbrücke und Rücklaufteiler, 20 cm Füllkörperkolonne sowie Rührer, Tauchrohr, Tropftrichter und Thermometer werden 679 g (3,5 Mol) Dimethylterephthalat (Fa. Oxxynova), 1,7 g Tetrabutyl-orthotitanat (0,25 Massen-% bezogen auf DMT, Fa. DuPont, Tyzor TnBT) und zunächst 200 g von insgesamt 748 g (8,5 Mol) n-Pentanol (> 99 % Reinheit, Fa. FLUKA) vorgelegt und langsam aufgeheizt, bis das DMT mit dem Alkohol eine homogene Mischung ergibt, dann erst wird der Rührer zugeschaltet. Der Rücklaufteiler wurde so eingestellt, dass die Kopftemperatur bei ca. 65 °C (Siedepunkt Methanol) konstant blieb. Ab einer Sumpftemperatur von ca. 185 °C wurde langsam der restliche Alkohol so zugefahren, dass die Temperatur im Kolben nicht unter 180 °C abfiel und ein für die Destillation ausreichender Rückfluss erhalten blieb. Das abgenommene Destillat wurde mittels GC-Analyse untersucht, um die abgetrennte Methanol-Menge zu bestimmen. Nach Abnahme eines Großteils des Methanols (ca. 90 % der zu erwartenden 7 Mol Methanol) wurde alle 30 Minuten jeweils eine Probe aus dem Reaktionskolben genommen und mittels GC analysiert. Die Umesterung war abgeschlossen, als der Gehalt an Dimethylester und Mischester (Methyl-Pentyl) insgesamt unter 0,3 Massen-% gefallen war (ca. 8 bis 9 h).

Zur Aufarbeitung wurde der Rücklaufteiler vollständig geöffnet und vorsichtig Vakuum (ca. 800 bis 5 hPa) angelegt, bis der Alkoholüberschuss abdestilliert war. Danach wurde auf 80 °C abgekühlt und die Apparatur mit Stickstoff belüftet. Vom Kolbeninhalt wurde die Säurezahl nach DIN EN ISO 2114 gemessen und mit der dreifachen stöchiometrischen Menge (in Bezug auf die Säurezahl) an 10 Massen-%iger wässriger Natronlauge 30 Minuten bei 80 °C gerührt. Anschließend wurde auf 160 °C aufgeheizt und ein Unterdruck von ca. 5 bis 30 hPa angelegt, bei dem über den Tropftrichter vorsichtig Wasser (8 Massen-% bezogen auf Kolbeninhalt) zugetropft wurde. Durch diese Prozedur (vgl. Wasserdampfdestillation) wurden weitere Leichtsieder abgetrennt. Anschließend wurde die Heizung abgestellt und das Produkt bei einem Druck von ca. 5 hPa getrocknet. Nachdem die Temperatur unter 100 °C gefallen war, wurde das Produkt über eine Nutsche mit Filterpapier und Filterhilfsmittel (Perlite) filtriert.

### Beispiel 2: Herstellung von Di-n-butylterephthalat

In einen 2-Liter-Mehrhalskolben mit Destillationsbrücke und Rücklaufteiler, 20 cm Füllkörperkolonne sowie Rührer, Tauchrohr, Tropftrichter und Thermometer werden 679 g (3,5 Mol) Dimethylterephthalat (Fa. Oxxynova), 1,7 g Tetrabutyl-orthotitanat (0,25 Massen-% bezogen auf DMT, Fa. DuPont, Tyzor TnBT)) und zunächst 200 g von insgesamt 629 g (8,5 Mol) n-Butanol (Fa. European Oxo, Reinheit 99,9 %) vorgelegt und langsam aufgeheizt, bis das DMT mit dem Alkohol eine homogene Mischung ergab, dann erst wurde der Rührer zugeschaltet. Der Rücklaufteiler wurde so eingestellt, dass die Kopftemperatur bei ca. 65 °C (Siedepunkt Methanol) konstant blieb. Ab einer Sumpftemperatur von ca. 185 °C wurde langsam der restliche Alkohol so zugefahren, dass die Temperatur im Kolben nicht unter 180 °C abfiel und ein ordentlicher Rückfluss erhalten blieb. Das abgenommene Destillat wurde mittels GC-Analyse untersucht, um die abgetrennte Methanol-Menge zu bestimmen. Nach Abnahme eines Großteils des Methanols (ca. 90 % der zu erwartenden 7 Mol Methanol) wurde alle 30 Minuten jeweils eine Probe aus dem Reaktionskolben genommen und mittels GC analysiert. Die Umesterung war abgeschlossen, als der Gehalt an Dimethylester und Mischester (Methyl-Butyl) insgesamt unter 0,3 % gefallen war (nach ca. 8 bis 9 h).

Zur Aufarbeitung wurde der Rücklaufteiler vollständig geöffnet und vorsichtig Vakuum (ca. 800 bis 5 hPa) angelegt, bis der Alkoholüberschuss abdestilliert war. Danach wurde auf 80 °C abgekühlt und die Apparatur mit Stickstoff belüftet. Vom Kolbeninhalt wurde die Säurezahl nach DIN EN ISO 2114 gemessen und mit der dreifachen stöchiometrischen Menge (in Bezug auf die Säurezahl) an 10 Massen-%iger wässriger Natronlauge 30 Minuten bei 80 °C gerührt. Anschließend wurde auf 160 °C aufgeheizt und ein Unterdruck von ca. 5 bis 30 hPa angelegt, bei dem über den Tropftrichter vorsichtig Wasser (8 Massen-% bezogen auf Kolbeninhalt) zugetropft wurde. Durch diese Prozedur (vgl. Wasserdampfdestillation) wurden weitere Leichtsieder abgetrennt. Anschließend wurde die Heizung abgestellt und das Produkt bei einem Druck von schließlich 5 hPa getrocknet. Nachdem die Temperatur unter 100 °C gefallen war, wurde das Produkt über eine Nutsche mit Filterpapier und Filterhilfsmittel (Perlite) filtriert.

### Beispiel 3: Herstellung von Plastisolen

Die verwendeten Einwaagen der Komponenten für die verschiedenen Plastisole 1 bis 3 sind der nachfolgenden Tabelle 1 zu entnehmen.

**Tabelle 1: Rezepturen (Alle Angaben in phr (= Massenteile pro 100 Massenteile PVC))**

| | Plastisol 1 (Vergleichs -plastisol) | Plastisol 2 (Vergleichs -plastisol) | Plastisol 3 (erfindungsgemäß) |
|---|---|---|---|
| Vestolit B 7021 (Fa. Vestolit) | 100 | 100 | 100 |
| Vestinol 9 (Fa. OXENO) | 30 | 30 | 30 |
| Diisobutylphthalat (Palatinol IC, BASF) | 20 | | |
| Di-n-butylterephthalat | | 20 | |
| Di-n-pentylterephthalat | | | 20 |
| Epoxidiertes Sojabohnenöl | 3 | 3 | 3 |
| Mark CZ 140 | 1,5 | 1,5 | 1,5 |

Die Weichmacher wurden vor der Zugabe auf 25 °C temperiert. Zuerst wurden die flüssigen Bestandteile und dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Beispiel 4: Messung der Plastisolviskositäten und der Lagerstabilität

Die Messung der Viskositäten der in Beispiel 3 hergestellten Plastisole wurden in Anlehnung an die DIN 53 019 mit einem Rheometer Physica DSR 4000 (Fa. Paar-Physica), welches über die zugehörige Software US 200 gesteuert wird, wie folgt durchgeführt.
Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o. g. Software. Folgende Punkte wurden angesteuert:
- Eine Vorscherung von 100 s⁻¹ für den Zeitraum von 60 s, bei der keine Messwerte aufgenommen wurden
- Eine Abwärtsrampe, beginnend bei 200 s⁻¹ bis herunter zu 0,1 s⁻¹, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h und 7 d durchgeführt. Zwischen diesen Zeitpunkten wurde die Paste bei 25 °C gelagert.

In der nachfolgenden Tabelle 2 sind für die Schergeschwindigkeit von 118 s⁻¹ jeweils die nach den angegebenen Lagerzeiten erhaltenen entsprechenden Viskositätswerte aufgeführt.

**Tabelle 2: Schergeschwindigkeit 118 s⁻¹ (Angaben der Viskositäten in Pa*s)**

| Plastisol | 1 | 2 | 3 |
|---|---|---|---|
| 2 h | 6,1 | 4,6 | 4,1 |
| 7 d | 14,6 | 9,6 | 8,0 |
| Anstieg um Faktor | 2,4 | 2,1 | 2 |

Die Viskositäten des erfindungsgemäßen Plastisols liegt in ihrem Niveau tiefer als die Vergleichsbeispiele. Außerdem ist der relative Anstieg der Viskosität bei dem erfindungsgemäßen Beispiel deutlich geringer und die Lagerstabilität somit höher.

### Beispiel 5: Messung des Gelierverhaltens

Die Untersuchung des Gelierverhaltens der Plastisole wurde in einem Oszillationsviskosimeter der Marke Bohlin CVO (Meßsystem PP20), welches schubspannungsgesteuert betrieben wurde, vorgenommen.

### Folgende Parameter wurden eingestellt:

Modus: Temperatur-Gradient
Start-Temperatur: 25 °C
End-Temperatur: 180 °C
Heiz/Kühlrate: 2 °C/min
Temperatur nach der Messung: 25 °C
Oszillations-Frequenz: 2 Hz
Verzögerungszeit: 1 s
Wartezeit: 15 s
Kontinuierliche Oszillation: an
Automatische Schubspannungsvorgabe: an
Startschubspannung: 0,3 Pa
Soll-Deformation: 0,002
Spaltweite 0,5 mm

### Durchführung der Messung:

Auf die untere Meßsystemplatte wurde mit dem Spatel ein Tropfen des zu messenden Plastisols (Rezepturen) luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Schutzabdeckung, die auch der Wärmeisolierung dient, aufgelegt und die Messung gestartet.

Aufgetragen wurde die sog. komplexe Viskosität des Plastisols in Abhängigkeit von der Temperatur. Ein Einsetzen des Geliervorganges ist in einem plötzlichen starken Anstieg der komplexen Viskosität zu erkennen. Je früher dieser Viskositätsanstieg einsetzt, desto besser ist die Gelierfähigkeit des Systems.

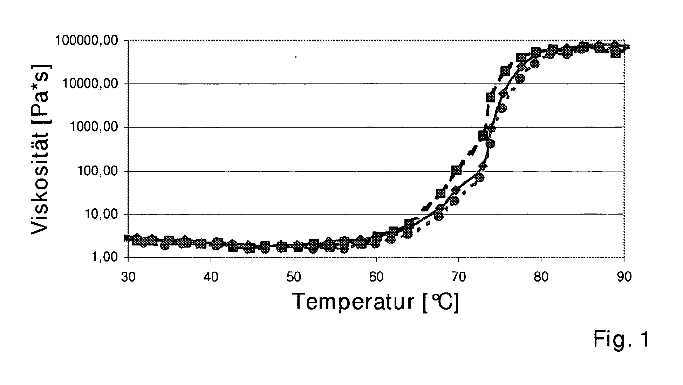

In Fig. 1 ist der für das Einsetzen der Gelierung relevante Ausschnitt des Viskositäts-Temperatur-Verlaufs ("Gelierkurve") für die in Beispiel 5 durchgeführten Messungen abgebildet. Die Y-Achse zeigt die komplexen Viskositäten in Pa.s, die X-Achse die Temperaturen in °C. Die Gelierkurve für Plastisol 1 ist als durchgezogene Linie dargestellt, wobei Rauten die einzelnen Messwerte darstellen. Die Gelierkurve für Plastisol 2 ist als gestrichelte Linie dargestellt, wobei Quadrate die einzelnen Messwerte darstellen. Die Gelierkurve für Plastisol 3 ist als punktierte Linie dargestellt, wobei Kreise die einzelnen Messwerte darstellen.

Das eine Vergleichsbeispiel (Plastisol 2) zeigt gegenüber dem anderen Vergleichsbeispiel (Plastisol 1) eine deutlich verbesserte Gelierung, die sich über einen bei tieferen Temperaturen einsetzenden Anstieg der Kurve ausdrückt. Das erfindungsgemäße Plastisol 3 ist im Rahmen der Messgenauigkeit bezüglich der Geliereigenschaften als etwa gleichwertig mit Plastisol 1 einzustufen.

Insgesamt wird deutlich, dass die erfindungsgemäßen Weichmacher und die daraus hergestellten erfindungsgemäßen Plastisole ein sehr gutes Eigenschaftsprofil aufweisen.

## Patentansprüche

1. Weichmacherzusammensetzungen, enthaltend Terephthalsäuredialkylester, in denen die Alkylreste eine längste Kohlenstoffkette von zumindest 4 Kohlenstoffatomen und eine Gesamtzahl an Kohlenstoffatomen pro Alkylrest von 5 aufweisen, und zumindest einen primären Weichmacher, ausgewählt aus den Phthalsäuredialkylestern, Trimellitsäuretrialkylestern, Adipinsäuredialkylestern, Terephthalsäuredialkylestern, 1,2-Cyclohexandisäurealkylestern, 1,3-Cyclohexandisäure-alkylestern und 1,4-Cyclohexandisäurealkylestern mit Alkyl = Alkylrest mit 8 bis 10 Kohlenstoffatomen, Glykoldibenzoaten und den Alkylsulfonsäureesterri von Phenol mit Alkyl = Alkylrest mit 8 bis 22 Kohlenstoffatomen sowie den acylierten und nicht acylierten Citronensäuretrialkylestem, Polymerweichmachern und Glycerinestern.

2. Weichmacherzusammensetzungen Anspruch 1,
**dadurch gekennzeichnet,**
**dass** die Alkylreste der Terephthalsäuredialkylester zu mehr als 60 % n-Pentyl-Reste sind.

3. Weichmacherzusammensetzungen nach Anspruch 1 oder 2,
**dadurch gekennzeichnet,**
**dass** die Alkylreste der Terephthalsäuredialkylester von 70 bis 99,9 % n-Pentyl-Reste und von 30 bis 0,1 % Methylbutyl-Reste sind.

4. Weichmacherzusammensetzungen nach Anspruch 3,
**dadurch gekennzeichnet,**
**dass** die Alkylreste der Terephthalsäuredialkylester von 85 bis 98 % n-Pentyl-Reste und von 15 bis 2 % Methylbutyl-Reste sind.

5. Weichmacherzusammensetzungen nach den Ansprüchen 1 bis 4,
**dadurch gekennzeichnet,**
**dass** der Anteil an primären Weichmachern von 20 bis 99 Massen-% beträgt.

6. Weichmacherzusammensetzungen nach zumindest einem der Ansprüche 1 bis 5,
**dadurch gekennzeichnet,**
**dass** sie zusätzlich zumindest einen Benzoesäurealkylester mit Alkyl = Alkylrest mit 7 bis 13 Kohlenstoffatomen aufweisen.

7. Weichmacherzusammensetzungen nach Anspruch 6,
**dadurch gekennzeichnet,**
**dass** Benzoesäurealkylester Benzoesäureisononylester ist.

8. Verwendung der Weichmacherzusammensetzungen gemäß den Ansprüchen 1 bis 7 als Weichmacher in Zusammensetzungen enthaltend ein Polymer, ausgewählt aus PVC, PVB, PAMA oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril, cyclischen Olefinen, oder in Klebstoffen, Dichtungsmassen, Lacken oder Farben.

9. Zusammensetzung enthaltend eine Weichmacherzusammensetzung gemäß den Ansprüchen 1 bis 7 und ein Polymer, ausgewählt aus PVC, PVB, PAMA oder Homo- oder Copolymere auf Basis von Ethylen, Propylen, Butadien, Vinylacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, cyclischen Olefinen.

10. Zusammensetzung nach Anspruch 9,
**dadurch gekennzeichnet,**
**dass** sie als PVC-Typ Suspensions-, Masse-, Mikrosuspensions- oder Emulsions-PVC enthält.

11. Zusammensetzung nach einem der Ansprüche 9 oder 10,
**dadurch gekennzeichnet,**
**dass** sie weitere Weichmacher, Füllstoffe, Pigmente, Stabilisatoren, Gleitmittel, Treibmittel, Kicker, Antioxidanzien oder Biozide enthält.

12. Zusammensetzung nach einem der Ansprüche 9 bis 11,
**dadurch gekennzeichnet,**
**dass** sie ein Plastisol, ein Kunstleder, ein Fußbodenbelag, ein Unterbodenschutz, ein beschichtetes Gewebe, eine Tapete, ein Lack, eine Farbe, eine Dichtungsmasse, eine Tinte oder ein Klebstoff ist.

## Claims

1. Plasticizer compositions comprising terephthalic acid dialkyl esters in which the alkyl radicals have a longest carbon chain of at least 4 carbon atoms and a total number of carbon atoms per alkyl radical of 5, and at least one primary plasticizer selected from phthalic acid dialkyl esters, trimellitic acid trialkyl esters, adipic acid dialkyl esters, terephthalic acid dialkyl esters, 1,2-cyclohexanedioic acid alkyl esters, 1,3-cyclohexanedioic acid alkyl esters and 1,4-cyclohexanedioic acid alkyl esters with alkyl = alkyl radical having 8 to 10 carbon atoms, glycol dibenzoates and the alkylsulphonic esters of phenol with alkyl = alkyl radical having 8 to 22 carbon atoms, and also acylated and non-acylated citric acid trialkyl esters, polymer plasticizers and glycerol esters.

2. Plasticizer compositions according to Claim 1, **characterized in that** more than 60% of the alkyl radicals of the terephthalic acid dialkyl esters are n-pentyl radicals.

3. Plasticizer compositions according to Claim 1 or 2, **characterized in that** from 70% to 99.9% of the alkyl radicals of the terephthalic acid dialkyl esters are n-pentyl radicals and from 30% to 0.1% are methylbutyl radicals.

4. Plasticizer compositions according to Claim 3, **characterized in that** from 85% to 98% of the alkyl radicals of the terephthalic acid dialkyl esters are n-pentyl radicals and from 15% to 2% are methylbutyl radicals.

5. Plasticizer compositions according to any of Claims 1 to 4, **characterized in that** the fraction of primary plasticizers is from 20% to 99% by mass.

6. Plasticizer compositions according to at least one of Claims 1 to 5, **characterized in that** they further contain at least one benzoic acid alkyl ester with alkyl = alkyl radical having 7 to 13 carbon atoms.

7. Plasticizer compositions according to Claim 6, **characterized in that** benzoic acid alkyl ester is isononyl benzoate.

8. Use of the plasticizer compositions according to any of Claims 1 to 7 as plasticizers in compositions comprising a polymer selected from PVC, PVB, PAMA or homopolymers or copolymers based on ethylene, propylene, butadiene, vinyl acetate, glycidyl acrylate, glycidyl methacrylate, methacrylates, acrylates, acrylates or methacrylates in which attached to the oxygen atom of the ester group there are alkyl radicals of branched or unbranched alcohols having one to ten carbon atoms, and styrene, acrylonitrile and cyclic olefins, or in adhesives, sealing compounds, varnishes or paints.

9. Composition comprising a plasticizer composition according to any of Claims 1 to 7 and a polymer selected from PVC, PVB, PAMA or homopolymers or copolymers based on ethylene, propylene, butadiene, vinyl acetate, glycidyl acrylate, glycidyl methacrylate, methacrylates, acrylates, acrylates in which attached to the oxygen atom of the ester group there are alkyl radicals of branched or unbranched alcohols having one to ten carbon atoms, and styrene, acrylonitrile and cyclic olefins.

10. Composition according to Claim 9, **characterized in that** it comprises suspension PVC, bulk PVC, microsuspension PVC or emulsion PVC as its PVC type.

11. Composition according to either of Claims 9 and 10, **characterized in that** it comprises further plasticizers, fillers, pigments, stabilizers, lubricants, blowing agents, kickers, anti-oxidants or biocides.

12. Composition according to any one of Claims 9 to 11, **characterized in that** it is a plastisol, a synthetic leather, a floorcovering, an underbody sealant, a coated fabric, a wallcovering, a varnish, a paint, a sealing compound, an ink or an adhesive.

## Revendications

1. Compositions de plastifiants, contenant des téréphtalates de dialkyle dans lesquels les radicaux alkyle comportent une chaîne carbonée la plus longue d'au moins 4 atomes de carbone et un nombre total d'atomes de carbone par radical alkyle égal à 5, et au moins un plastifiant primaire choisi parmi les phtalates de dialkyle, les trimellitates de trialkyle, les adipates de dialkyle, les téréphtalates de dialkyle, les 1,2-cyclohexanedioates d'alkyle, les 1,3-cyclohexanedioates d'alkyle et les 1,4-cyclohexanedioates d'alkyle, où alkyle = radical alkyle ayant de 8 à 10 atomes de carbone, les dibenzoates de glycol et les esters alkylsulfoniques de phénol où alkyle = radical alkyle ayant de 8 à 22 atomes de carbone, ainsi que les citrates de trialkyle acylés ou non acylés, des plastifiants polymères et des esters de glycérol.

2. Compositions de plastifiants selon la revendication 1, **caractérisées en ce que** les radicaux alkyle des téréphtalates de dialkyle sont à raison de plus de 60 % des radicaux n-pentyle.

3. Compositions de plastifiants selon la revendication 1 ou 2, **caractérisées en ce que** les radicaux alkyle des téréphtalates de dialkyle sont à raison de 70 à 99,9 % des radicaux n-pentyle et à raison de 30 à 0,1 % des radicaux méthylbutyle.

4. Compositions de plastifiants selon la revendication 3, **caractérisées en ce que** les radicaux alkyle des téréphtalates de dialkyle sont à raison de 85 à 98 % des radicaux n-pentyle et à raison de 15 à 2 % des radicaux méthylbutyle.

5. Compositions de plastifiants selon les revendications 1 à 4, **caractérisées en ce que** la teneur en plastifiants primaires va de 20 à 99 % en masse.

6. Compositions de plastifiants selon au moins l'une des revendications 1 à 5, **caractérisées en ce qu'**elles comportent au moins un benzoate d'alkyle où alkyle = radical alkyle ayant de 7 à 13 atomes de carbone.

7. Compositions de plastifiants selon la revendication 6, **caractérisées en ce que** le benzoate d'alkyle est le benzoate d'isononyle.

8. Utilisation des compositions de plastifiants selon les revendications 1 à 7, en tant que plastifiant dans des compositions contenant un polymère choisi parmi le PVC, le PVB, le PAMA ou des homo- ou copolymères à base d'éthylène, propylène, butadiène, acétate de vinyle, acrylate de glycidyle, méthacrylate de glycidyle, méthacrylates, acrylates, acrylates ou méthacrylates à radicaux alkyle, liés à l'atome d'oxygène du groupe ester, d'alcools ramifiés ou non ramifiés comportant de un à dix atomes de carbone, de styrène, d'acrylonitrile, d'oléfines cycliques, ou dans des adhésifs, des matériaux d'étanchéité, des vernis ou des peintures.

9. Composition contenant une composition de plastifiant selon les revendications 1 à 7 et un polymère choisi parmi le PVC, le PVB, le PAMA ou des homo- ou copolymères à base d'éthylène, propylène, butadiène, acétate de vinyle, acrylate de glycidyle, méthacrylate de glycidyle, méthacrylates, acrylates, acrylates à radicaux alkyle, liés à l'atome d'oxygène du groupe ester, d'alcools ramifiés ou non ramifiés comportant de un à dix atomes de carbone, de styrène, d'acrylonitrile, d'oléfines cycliques.

10. Composition selon la revendication 9, **caractérisé en ce qu'**elle contient en tant que type de PVC du PVC en suspension, en masse, en microsuspension ou en émulsion.

11. Composition selon la revendication 9 ou 10, **caractérisé en ce qu'**elle contient d'autres plastifiants, des charges, des pigments, des stabilisants, des lubrifiants, des agents porogènes, des amorceurs, des antioxydants ou des biocides.

12. Composition selon l'une quelconque des revendications 9 à 11, **caractérisé en ce qu'**elle est un Plastisol, un cuir synthétique, un revêtement de sol, une protection de base de caisse, un tissu revêtu, une tenture, un vernis, une peinture, un matériau d'étanchéité, une encre ou un adhésif.
